# EUROPEAN PATENT SPECIFICATION

(11) **EP 1 107 731 B1**
(45) Date of publication and mention of the grant of the patent: **06.11.2002**
(21) Application number: 99968213.1
(22) Date of filing: 30.08.1999
(51) Int. Cl.: A61K 9/00, A61K 47/24

(54) **SYNTHETIC TEAR FLUID**
SYNTHETISCHE TRÄNENFLÜSSIGKEIT
LIQUIDE LACRYMAL DE SYNTHESE

(30) Priority: 28.08.1998 EP 98116291
(43) Date of publication of application: 20.06.2001
(73) Proprietor: Stoffel, Wilhelm, Prof. Dr., 50933 Köln (DE)
(72) Inventor: Stoffel, Wilhelm, Prof. Dr., 50933 Köln (DE)
(74) Representative: Meyers, Hans-Wilhelm, Dr.Dipl.-Chem.
(86) International application number: EP9906370
(87) International publication number: WO00012061

(56) References cited:
- WO-A-90/11781
- WO-A-91/12808
- WO-A-94/04155
- US-A- 4 755 388

## Description

The invention is related to a synthetic tear fluid.

The proper functioning of the lacrimal apparatus is determined by the chemical properties of the constituents of the tear film system and its stability. The tear film remains continuous between blinks and fulfils the function of forming an optically smooth surface and a refractive medium (45-47 diopters). Furthermore, it maintains an epithelium tear interface, ensuring hydratization of the Cornea.

The tear film is composed of three layers:
1. the outermost monomolecular lipid film described as "waxy layer with cholesterylesters",
2. an aqueous mucin and protein containing layer, and
3. the gel like mucus layer.

The mean thickness of the film is in the range from 36-48 nm as determined in the normal open eye, and depends on the palpebral fissure width.

The principle of the formation of the film is
1. lids secrete the hydratable mucus layer (glycochalyx, 30 µm thickness),
2. the aqueous tears spread over the mucus layer, and
3. Meibomian oil covers the aqueous surface.

The lipid layer may vary in thickness and composition in normal individuals but also in chronic blepharitis which influences its properties. This might be of outmost relevance for the stability of the film and the development of dry eye syndrome.

A blink (8-10/min) is associated with the lipid discharge from the Meibomian glands. Mucus comes from global cells and is replaced every 30 minutes. Meibomian secretion forms a solid to semifluid sheath suspended from the upper lid, thickening as eye lid closes and thins out again on opening.

The function of the lipid film is
a) to reduce the evaporation of the aqueous tears which occurs at a normal rate of 0.085 to 1.7 µl/min. This evaporation grade is ten times higher in the absence of a lipid film.
b) to reduce the surface tension of the aqueous phase.

Disturbances of the lipid composition lead to mucin and fluid deficiency and cause dry eye syndrome. An overview is given in Brauninger, GF.E., Shah, D.O., Kaufman, H.E., "Direct physical demonstration of the oil layer on tear film surface", Am.J.Ophthalmol. 73, 132-134 (1972); and Kaercher, T., Möbius, D., Welt, R., "Biophysical characteristics of the Maibomian lipid layer under in vitro conditions", Intl.Ophthalmol. 16, 167-176 (1992).

US-A-4,755,388 discloses drugs encapsulated in liposomes, where the drugs are low molecular weight, negatively charged polar drugs and the liposomes are comprised of high transition temperature phospholipids and cholesterol.

WO-A-94/04155 discloses a dehydroepiandrosterone therapy for the treatment of eye disorders.

WO-A-91/12808 discloses an artificial tear composition for use in treating or preventing dry eye syndrome containing a phospholipid, and optionally hyaluronic acid or its salts in a suitable carrier.

WO-A-90/11781 discloses methods for delivering therapeutic agents to wounds in liposomes which preferentially bind to the wounds. Especially methods for delivering therapeutic agents in liposomes to wounds on the ocular surface of the eye are disclosed.

It is an object of the present invention to provide an aqueous, lipid-containing suspension having improved properties when used as a synthetic tear fluid. It can further be used for the application of therapeutic or cosmetic substances into the eye. Surprisingly, this object is achieved by a synthetic tear fluid having the features of claim 1.

The synthetic tear fluid of the present invention comprises glycerophospholipids. This composition spreads on the surface of the Cornea as a monolayer and shows substantial hysteresis of the pressure/area isotherms at a temperature of 20 to 40°C on compression and expansion below the collapse point of the monomolecular film, like the lipids do in the Langmuir experiment.

A glycerophospholipid contains two fatty acids, bound to the glycerol backbone. Typically, these fatty acids have an even number of carbon atoms, usually between 14 and 24. These carbon atoms can form branched or unbranched chains. The chains may have one or more double bonds, or may be fully saturated. The glycerophospholipids of the present invention can have the same or different fatty acids in one molecule, and the glycerophospholipids can be used as pure substances or as mixtures of glycerophospholipids.

In a preferred embodiment the glycerophospholipids have a low transition temperature especially a transition temperature below 37°C and more preferred below 30°C. It is preferred that the glycerophospholipids comprise unsaturated fatty acids because of their low transition temperature. Typical fatty acids are palmitate, oleate, and linoleate. Very preferred are dilinoleoyl phospholipids such as dilinoleoylphosphatidylcholin. Glycerophospholipids can be chemically synthesized or, more typically, extracted and purified from natural sources. Suitable glycerophospholipids are selected from phosphatidylcholines, phosphatidylserines, phosphatidylethanolamines, phosphatidylinositol, or diphosphatidylglycerol.

Furthermore, the synthetic tear fluid of the present invention further comprises cholesterol in a molar ratio up to 1:1 (glycerophospholipids:cholesterol).

The synthetic tear fluid further comprises a lipophilic anionic compound, such as gangliosides, sulfatides or chemically synthesized diacyllipids or dialkyllipids with anionic or cationic polar head groups. Preferably, these lipophilic anionic compounds are gangliosides. Gangliosides are derivatives of ceramide wherein one or more sugar moieties, e.g. glucose, galactose, N-acetylgalactosamine, or N-acetylneuraminic acid are fused to C-1 of sphingosine.

The composition further comprises water and a buffer substance.

In a preferred embodiment, the synthetic tear fluid of the present invention is isotonic. The buffer substance can be used to render the suspension isotonic. Furthermore, the composition used in the present invention may comprise a preservative, e.g. benzylalkonium chloride.

In a preferred embodiment, the synthetic tear fluid may comprise a natural lipid solubilizer. This solubilizer facilitates the formation of micelles in the aqueous, lipid-containing suspension of the present invention. Preferably, the molar ratio of natural lipid solubilizer:total lipids is from 1:10 to 1:100. Monoacylglycerol, monoalkylglycerol, monoacylglycol, monoalkylglycol, ceramide (N-acylsphingosine), psychosine (sphingosine 1-β-galactoside) and sphinosyl-phosphoryl-cholin are the preferred natural lipid solubilizers of the present invention. The concentration is preferably in the range of 0.5 to 10% (w/w) of the suspension.

The total concentration of the lipids in the synthetic tear fluid of the present invention is preferably in the range from 0.5 to 20 µmol/ml. A concentration of 0.5 to 5 µmol/ml is preferred.

In a preferred embodiment, the synthetic tear fluid further comprises sphingolipids in a glycerophospholipid:sphingolipid molar ratio of 20:1 to 2,5:1. Sphingolipids are derivatives of the amino-alcohol sphingosine. In general, sphingosine can form an amide with fatty acids to yield ceramide which may be linked via a phosphate group to choline, serine, ethanolamine, glycerol or inositol. The explanations of the fatty acids above apply for the sphingosine derivative as well.

Sphingomyelin is a preferred sphingolipid of the present invention. Sphingolipids can either be prepared by chemical synthesis or by extraction and purification from natural sources.

The synthetic tear fluid of the present invention can be prepared by a method comprising the steps of combining dried lipids with an aqueous phase. Preferably, the dried lipids are prepared by preparing stock solutions of the individual lipids in an organic solvent, combining the solutions of the lipids, evaporating the combined solutions to dryness, adding an aqueous, preferably buffered solution to give an emulsion and sonicating the emulsion or extruding the emulsion through filters of defined pore size to prepare multilamellar liposomes of homogenous size. Preferably the emulsion is vortexed before the extrusion and the extrusion process is repeated about 10 to 20 times. Suitable filters are polycarbonatfilters with pore sizes from 15 nm up to 5 µm.

The method can further comprise a sterilizing and/or filtration step and optionally adding a preservative.

The synthetic tear fluid of the present invention is especially useful for the treatment of Cornea sicca (dry eye syndrome), epithelial lesions, irritation of the Cornea, e.g. by contact lenses, as a carrier for the application of therapeutic and/or cosmetic substances, preferably antibiotics, antiviral drugs, cytostatic drugs, antimycotic drugs, wound healing substances, and diagnostic markers for Cornea lesions, into the eye.

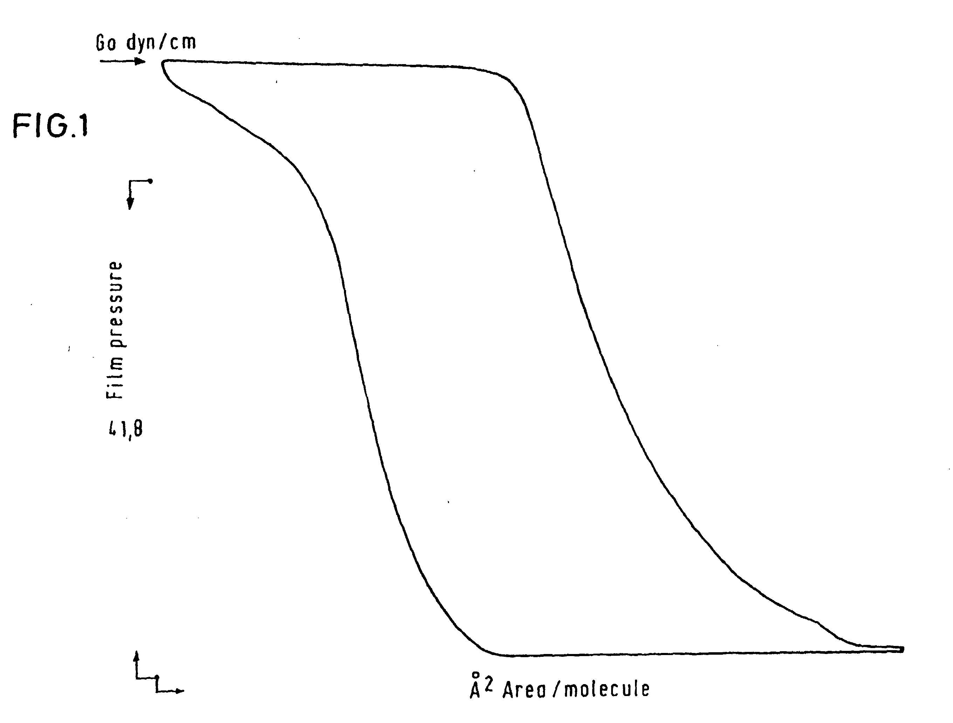

Figure 1 represents a typical F/A isotherm obtained according to example 1.

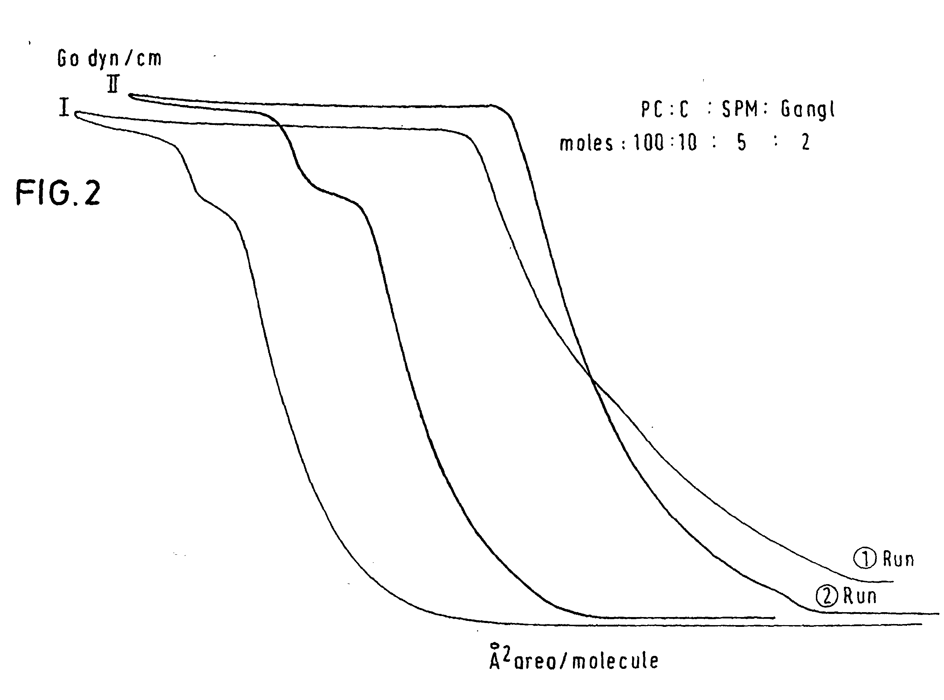

Figure 2 represents the F/A isotherm of lipid mixtures of example 2.

### Examples

### Example 1 (Langmuir Experiment)

Monomolecular lipid films were generated by spreading lipids on a water subphase of a Langmuir balance designed and provided by Bayer (Leverkusen, Germany), Department of Physics (Drs. Meskat and Sucker). It is a selfrecording instrument which correlates simultanously F (lateral pressure)/A (area) isotherms reflecting the lipid conformation and structures in the monomolecular layer. A trough is filled with water. A measuring barrier which picks up the pressure of the film compressed by a film compressing barrier. The area of the film at respective lateral (compression) pressure is recorded by a X/Y recorder. The trough with the aqueous subphase is temperature controlled. Lipid samples are applied by a micropipette which allows the accurate application of a lipid concentration. The F/A isotherm records the area per lipid molecule of single lipid classes or mixtures of different lipids at defined pressures, which includes phase transition temperatures, collapse point etc. The compression barrier can be geared in the film compression or film expansion mode, thereby probing for the hysteresis of the respective film.

### Example 2

PC (soya): Cholesterol:sphingomyelin:ganglioside 100:10:5:2 molar ratio

50 mg (60 µ moles) of PC (soya) is dissolved in 10 ml of chloroform and combined with solutions of
2.3 mg (6 µ moles) cholesterol in 0.6 ml of chloroform,
2.4 mg (3 µ moles) sphingomyelin in 0.3 ml of methanol/ chloroform (2:1, v/v), and
2 mg (1.2 µ moles) ganglioside in 0.5 ml of methanol/ chloroform (1:1, v/v).

The solution is evaporated under a stream of N₂ at a temperature of 30°C under reduced pressure. 5 ml of an aqueous solution containing sterilized PBS are added and the solution is vortexed and extruded 15 times through a 100 nm pore diameter polycarbonate membrane (Arestin, Inc. PO 8530, Ottawa, Canada) in the Liposo FAST™ stabilizer.

## Claims

1. Synthetic tear fluid comprising
- at least one glycerophospholipids selected from the group consisting of phosphatidylcholines, phosphatidylserines, phosphatidylethanolamines, and phosphatidylinositol,
- cholesterol in a glycerophospholipid:cholesterol molar ratio up to 1:1
- at least one lipophilic anionic compound, in a glycero-phospholipid:lipophilic anionic compound molar ratio of 100:1 to 10:1
- at least one buffer substance
- water.

2. Synthetic tear fluid according to claim 1 comprising a ganglioside as lipophilic anionic compound.

3. Synthetic tear fluid according to claim 1 or 2 further comprising sphingolipids in a glycerophospholipid:sphingolipid molar ratio of 20:1 to 2.5:1.

4. Synthetic tear fluid according to any one of claims 1 to 3, further comprising a natural lipid solubilizer in a molar ratio of natural lipid solubilizer:total lipids from 1:10 to 1:100.

5. Synthetic tear fluid according to claim 4, wherein the natural lipid solubilizer is selected from the group consisting of monoacylglycerol, monoalkylglycerol, monoacylglycol, monoalkylglycol, ceramide (N-acylsphingosine), psychosine (sphingosine 1-β-galactoside) and sphingosyl-phosphoryl-cholin.

6. Synthetic tear fluid according to any one of the claims 1 to 5, wherein the total concentration of lipids is from 0.5 to 20 µmol/ml.

7. Medicament comprising the synthetic tear fluid of any one of the claims 1 to 6.

8. Use of the synthetic tear fluid according to any one of the claims 1 to 6 as a carrier for the application of cosmetic substances.

9. Use of the synthetic tear fluid according to any one of the claims 1 to 6 for the preparation of a carrier for the application of therapeutic substances, preferably antibiotics, antiviral drugs, cytostatic drugs, antimycotic drugs, wound healing substances, and diagnostic markers for Cornea lesions.

10. Use of the synthetic tear fluid according to any one of the claims 1 to 6 for the preparation of a medicament for the treatment of Cornea sicca (dry eye syndrome), epithelial lesions, irritation of the Cornea, e.g. by contact lenses.

## Patentansprüche

1. Synthetische Tränenflüssigkeit, umfassend:
- wenigstens ein Glycerophospholipid, das aus der Gruppe ausgewählt ist, die aus Phosphatidylcholinen, Phosphatidylserinen, Phosphatidylethanolaminen und Phosphatidylinosit besteht;
- Cholesterin in einem Stoffmengenverhältnis von Glycerophospholipid zu Cholesterin von bis zu 1:1;
- wenigstens eine lipophile anionische Verbindung in einem Stoffmengenverhältnis von Glycerophospholipid zu lipophiler anionischer Verbindung von 100:1 bis 10:1;
- wenigstens eine Puffersubstanz;
- Wasser.

2. Synthetische Tränenflüssigkeit gemäß Anspruch 1, die ein Gangliosid als lipophile anionische Verbindung umfasst.

3. Synthetische Tränenflüssigkeit gemäß Anspruch 1 oder 2, die weiterhin Sphingolipide in einem Stoffmengenverhältnis von Glycerophospholipid zu Sphingolipid von 20:1 bis 2,5:1 umfasst.

4. Synthetische Tränenflüssigkeit gemäß einem der Ansprüche 1 bis 3, die weiterhin ein Solubilisator in Form eines natürlichen Lipids in einem Stoffmengenverhältnis von Solubilisator in Form eines natürlichen Lipids zu Gesamtlipiden von 1:10 bis 1:100 umfasst.

5. Synthetische Tränenflüssigkeit gemäß Anspruch 4, wobei der Solubilisator in Form eines natürlichen Lipids aus der Gruppe ausgewählt ist, die aus Monoacylglycerin, Monoalkylglycerin, Monoacylglycol, Monoalkylglycol, Ceramid (N-Acylsphingosin), Psychosin (Sphingosin-1-β-galactosid) und Sphingosylphosphorylcholin besteht.

6. Synthetische Tränenflüssigkeit gemäß einem der Ansprüche 1 bis 5, wobei die Gesamtkonzentration an Lipiden 0,5 bis 20 µmol/ml beträgt.

7. Medikament, das die synthetische Tränenflüssigkeit gemäß einem der Ansprüche 1 bis 6 umfasst.

8. Verwendung der synthetischen Tränenflüssigkeit gemäß einem der Ansprüche 1 bis 6 als Träger für die Anwendung kosmetischer Substanzen.

9. Verwendung der synthetischen Tränenflüssigkeit gemäß einem der Ansprüche 1 bis 6 zur Herstellung eines Trägers für die Anwendung therapeutischer Substanzen, vorzugsweise Antibiotika, Antivirenmedikamente, cytostatischer Medikamente, antimykotischer Medikamente, wundheilender Substanzen und diagnostischer Marker für Hornhautläsionen.

10. Verwendung der synthetischen Tränenflüssigkeit gemäß einem der Ansprüche 1 bis 6 zur Herstellung eines Medikaments für die Behandlung von Keratokonjunktivitis sicca (trockenes Auge; Sicca-Syndrom), Epithelläsionen, Irritationen der Cornea, z.B. durch Kontaktlinsen.

## Revendications

1. Liquide lacrymal synthétique comprenant
- au moins un glycérophospholipide choisi dans le groupe formé par les phosphatidylcholines, les phosphatidylsérines, les phosphatidyléthanolamines et le phosphatidylinositol,
- du cholestérol en un rapport molaire glycérophospholipide :cholestérol limité à 1:1
- au moins un composé anionique lipophile, en un rapport molaire glycérophospholipide :composé anionique lipophile de 100:1 à 10:
- au moins une substance tampon
- de l'eau.

2. Liquide lacrymal synthétique selon la revendication 1, comprenant un ganglioside en tant que composé anionique lipophile.

3. Liquide lacrymal synthétique selon la revendication 1 ou 2, comprenant de plus des sphingolipides en un rapport molaire glycérophospholipide:sphingolipide de 20:1 à 2,5:1.

4. Liquide lacrymal synthétique selon l'une quelconque des revendications 1 à 3, comprenant de plus un agent solubilisateur de lipide naturel en un rapport molaire agent solubilisateur de lipide naturel:lipides totaux de 1:10 à 1:100.

5. Liquide lacrymal synthétique selon la revendication 4, dans lequel l'agent solubilisateur de lipide naturel est choisi dans le groupe formé par un monoacylglycérol, un monoalkylglycérol, un monoacylglycol, un monoalkylglycol, un céramide (N-acylsphingosine), la psychosine (sphingosine 1-â-galactoside) et la sphingosylphosphoryl-choline.

6. Liquide lacrymal synthétique selon l'une quelconque des revendications 1 à 5, dans lequel la concentration totale de lipides est de 0,5 à 20 ìmol/ml.

7. Médicament comprenant le liquide lacrymal synthétique selon l'une quelconque des revendications 1 à 6.

8. Utilisation du liquide lacrymal synthétique selon l'une quelconque des revendications 1 à 6, comme véhicule pour l'application de substances cosmétiques.

9. Utilisation du liquide lacrymal synthétique selon l'une quelconque des revendications 1 à 6, pour la préparation d'un véhicule destiné à l'application de substances thérapeutiques, de préférence des antibiotiques, des médicaments antiviraux, des médicaments cytostatiques, des médicaments antimycosiques, des substances cicatrisantes et des marqueurs diagnostiques pour lésions de la cornée.

10. Utilisation du liquide lacrymal synthétique selon l'une quelconque des revendications 1 à 6, pour la préparation d'un médicament destiné au traitement de la cornea sicca (syndrome de l'oeil sec), de lésions épithéliales, d'une irritation de la cornée, par exemple par des lentilles de contact.
